# EUROPÄISCHE PATENTSCHRIFT

(11) **EP 4 463 446 B1**
(45) Veröffentlichungstag und Bekanntmachung des Hinweises auf die Patenterteilung: **21.10.2026**
(21) Anmeldenummer: 23701009.5
(22) Anmeldetag: 16.01.2023
(51) Int. Cl.: C07D 307/68, C07D 233/90, C07D 263/34, C07D 277/32, A61P 17/08, A61P 17/10, A61K 31/341

(54) **TOFA-ANALOGA, ZUBEREITUNGEN ZUR SEBUMREDUKTION MIT EINEM GEHALT AN SOLCHEN ANALOGA UND DIE KOSMETISCHE UND/ODER THERAPEUTISCHE VERWENDUNG SOLCHER ANALOGA ALS WIRKSAMES PRINZIP ZUR SEBUMREDUKTION ODER -VERHINDERUNG**
TOFA ANALOGUES, SEBUM REDUCTION PREPARATIONS CONTAINING SAID TYPE OF ANALOGUES AND THE COSMETIC AND/OR THERAPEUTIC USE OF SAID TYPE OF ANALOGUES AS AN EFFECTIVE PRINCIPLE FOR REDUCING OR PREVENTING SEBUM
ANALOGUES DE TOFA, PRÉPARATIONS POUR LA RÉDUCTION DE SÉBUM CONTENANT LEDIT TYPE D'ANALOGUES ET UTILISATION COSMÉTIQUE ET/OU THÉRAPEUTIQUE DUDIT TYPE D'ANALOGUES EN TANT QUE PRINCIPE EFFICACE POUR RÉDUIRE OU PRÉVENIR LE SÉBUM

(30) Priorität: 08.02.2022 DE 102022201276
(43) Veröffentlichungstag der Anmeldung: 20.11.2024
(60) Teilanmeldung: 25160774.3 / 4 552 638
(73) Patentinhaber: Beiersdorf AG, 22529 Hamburg (DE)
(72) Erfinder: SEIDEL, Judith, 13507 Berlin (DE); REUTER, Jörn Hendrik, 24558 Henstedt Ulzburg (DE); KAMAL, Ahmed, Hamburg 20255 (DE); SIMMERING, Annika, 20249 Hamburg (DE); MIELKE, Heiko, 21629 Neu Wulmstorf (DE)
(74) Vertreter: Beiersdorf AG
(86) Internationale Anmeldenummer: PCT/EP2023/050861
(87) Internationale Veröffentlichungsnummer: WO 2023/151897

(56) Entgegenhaltungen:
- WO-A1-2011/005660
- DE-A1- 4 033 563
- BAROUDI ET AL.: "Olumacostat glasaretil. Acetyl-CoA carboxylase (ACC) inhibitor, Acne therapy", DRUGS OF THE FUTURE, vol. 43, no. 3, 2018, ES, pages 175 - 179, XP093024230, ISSN: 0377-8282, DOI: 10.1358/dof.2018.043.03.2766398

## Beschreibung

Die vorliegende Erfindung betrifft neue Substanzen und deren Verwendungen als Wirkstoffe und Zubereitungen, solche Wirkstoffe enthaltend, welche gegen fettige und/oder unreine Haut, sowie milden Formen der Akne und/oder bei fettigem Haar wirksam sind.

Seborrhö ist eine gesteigerte Funktion der Talgdrüsen durch Veranlagung. Sowohl Kopfhaut als auch Gesichtshaut erscheinen fettig. Die Zusammensetzung des seborrhöischen Sebums ist gegenüber dem normalen Sebum verändert. man unterscheidet 3 Entwicklungsstufen der Seborrhö:
1. Einfache Seborrhö: Leichte Fälle, fettig nach 8 Tagen.
2. Ölige Seborrhö: Bereits nach 2-3 Tagen fettig.
3. Irreversible Form: Nicht mehr umkehrbar. Die Seborrhö, bei der das Haar bereits nach einem Tag wie in Fett gebadet aussieht.

Die übermäßige Absonderung der Talgdrüsen kann unter anderem durch androgenetische Störungen (männliche Geschlechtshormonstörung) ausgelöst werden und hat einen ästhetischen Nachteil auf das Gesamtbild der Haare. Diese Störung kann auch die Ursache für auftretenden Haarausfall sein. Vorläufer ist jeweils der seborrhöische Zustand der Kopfhaut. Vegetative Störungen sowie unsachgemäße Pflege können das Hautbild und auch den Haarzustand noch verschlechtern. Auch bei Seborrhö kann das Haar selbst durch Störungen im Keratinaufbau trocken sein. Trockenes, angegriffenes Haar wird häufig hervorgerufen durch äußere Beanspruchung wie z. B. Sonne oder chemischen Behandlungen. Zu heißes Föhnen oder nicht richtige Pflege von angegriffenem Haar können zu Schädigungen führen.

Die Ursachen für fettiges Haar liegen im Körper des Menschen und sind hormonell bedingt. Jedes Haar besitzt eine eigene Talgdrüse, die Fett (auch Sebum oder Talg genannt) produziert. Die Sebum-Produktion wird hormonell gesteuert, und es kann je nach Hormonempfindlichkeit der Talgdrüse zu einer Über- oder Unterproduktion kommen. Der Talg selbst hat die Funktion die Kopfhaut geschmeidig zu halten. Er gelangt aus der Talgdrüse auf die Kopfhaut und erst später den Haaransatz. Dort wird er normalerweise vom Haarschaft aufgenommen und bleibt unsichtbar. Bei einer Talgüberproduktion ist der Haarschaft nicht mehr in der Lage diesen aufzunehmen. Er wird als Fettfilm am Haar sichtbar. Die Folge ist strähniges, fettig glänzendes Haar.

Dadurch dass die Talgdrüsenproduktion vom Hormonhaushalt abhängig ist, lässt sich das Problem fettigen Haares nicht grundsätzlich lösen, denn die Talgdrüsen produzieren stetig Fett. Konsequente Pflege und hochwertige Pflegeserien sind immer noch die beste Methode zur Bekämpfung fettigen Haares.

Leider hat fettiges Haar sehr lästige Auswirkungen. Die Haare werden schon kurze Zeit nach dem Waschen wieder strähnig und die Frisur hält nicht.

Entgegen landläufiger Meinung ist es nur ein Gerücht, dass durch zu häufiges Waschen das Haar noch schneller fettig wird. Milde Shampoos gegen fettiges Haar sorgen dafür, dass übermäßiges Fett entfernt wird. Haar und Kopfhaut werden mit ausreichend Feuchtigkeit versorgt und gleichen die Überproduktion der Talgdrüsen aus.

Fettiges Haar und Schuppenbildung gehören zu den häufigsten Haarproblemen. Diese Anomalien sind auf eine Störung der Talgdrüsentätigkeit zurückzuführen. Bei einer Überfunktion der Talgdrüsen sprechen wir von einer Seborrhö. Dabei sind zwei Formen zu unterscheiden: die ölige Form (Seborrhoea oleosa) und die trockene Form (Seborrhoea sicca).

### Die Seborrhoea oleosa:

Hier liegt eine Überfunktion der Talgdrüsen vor, bei der die Talgdrüsen zu viel und zu öligen Talg erzeugen. Die Haut zeigt deshalb einen fettigen Glanz, und die Haare sind schon 2 bis 3 Tage nach der Wäsche wieder bis in die Spitzen fettig und strähnig.

### Die Seborrhoea sicca:

Sie ist ebenfalls auf eine Überfunktion der Talgdrüsen zurückzuführen, aber der Hauttalg ist trockener, er hat eine festere Konsistenz. Mit den Schüppchen der Oberhaut bildet er große, leicht zerreibbare Talgschuppen. Die Kopfhaut zeigt einen wachsartigen Glanz, das Haar fettet nur am Ansatz nach, die Längen und besonders die Spitzen sind trocken und sogar spröde.

Zur Behandlung der Seborrhö gehört zunächst mal die regelmäßige und gründliche Kopfwäsche mit Spezialshampoos, die so oft durchgeführt werden kann, wie es nötig erscheint. Die Wäsche sollte mit einer Massage im Bindegewebe verbunden sein, weil dadurch dir Talgdrüsen stärker entleert werden, was die Nachfettung verzögert.

Bei der unreinen Haut sowie bei milden Formen der Akne sind neben einer verstärkten Sebumproduktion auch andere Einflüsse wie z. B. bakterielle Sekundärinfektionen von ätiologischer Bedeutung. Einer der wichtigsten Mikroorganismen, der in Zusammenhang mit unreiner Haut steht, ist Propionibacterium acnes.

Unreine Haut und/oder Komedonen beeinträchtigen das Wohlbefinden der Betroffenen aber selbst in leichten Fällen. Da praktisch jeder oder jede Jugendliche von unreiner Haut irgendeiner Ausprägung betroffen ist, besteht bei vielen Personen Bedarf, diesem Zustande abzuhelfen. Da ein wesentlicher Faktor dabei die übermäßige Talgproduktion ist, kann in vielen Fällen bereits durch den Einsatz von sebumreduzierenden Wirkstoffen eine Verbesserung des Hautzustandes erreicht werden.

Aufgabe der vorliegenden Erfindung war es also, gegen fettige und/oder unreine Haut, milde Formen der Akne sowie gegen fettiges Haar, wirksame Zubereitungen zu finden.

Der Stand der Technik kennt bereits einige Versuche zur Lösung dieses Problems, die jedoch nicht alle Vorteile der hier vorgestellten Entwicklung kennen.

Die WO 2011/005660 offenbart Analoga von 5-(Tetradecyloxy)-2-furancarbonsäure (TOFA) und deren antiseborrhöische Wirkung.

Die Schrift WO 2018/022797 offenbart Analoga von 5-(Tetradecyloxy)-2-furancarbonsäure (TOFA) und deren Wirkung gegen Acne Vulgaris.

Die Schrift WO 2019/115405 offenbart Analoga von Pyrrolderivaten und deren antiseborrhöische Wirkung.

Es wurde aber überraschend gefunden, und darin liegt die Lösung der Aufgabe, dass neue Verbindungen der allgemeinen Formel (1) 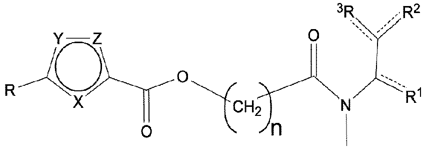
bei denen R = n-Tetradecyl bedeutet,
n = eine Zahl von 1 bis 5 bedeutet
X = O, N, N-H oder S bedeutet,
Y und Z unabhängig voneinander bedeuten C-H, N, N-H, O oder S, wobei die Gruppen so gewählt werden, dass der Fünfring Hückel-aromatischen Charakter hat,
dass von R² und R³ nicht gleichzeitig eine Doppelbindung ausgehen kann,
und R¹, R² und R³ unabhängig voneinander bedeuten -H, eine Oxogruppe, -C₁-C₂₄-Alkyl (linear und verzweigt), -C₁-C₂₄-Alkenyl (linear und verzweigt), -C₁-C₈-Cycloalkyl, -C₁-C₈-Cycloalkyl-Alkylhydroxy, mit einer oder mehreren Hydroxylgruppen und/oder mit einer oder mehreren primären, sekundären und/oder tertiären Aminogruppen substituiertes -C₁-C₂₄ Alkyl (linear und verzweigt) und/oder mit einer oder mehreren gegebenenfalls veresterten Carboxylgruppen substituiertes -C₁-C₂₄ Alkyl (linear und verzweigt), -C₁-C₂₄-Alkylaryl-Alkyl-Hydroxy (linear und verzweigt), -C₁-C₂₄-Alkylheteroaryl (linear und verzweigt), -C₁-C₂₄-Alkyl-O-C₁-C₂₄-Alkyl (linear und verzweigt), -C₁-C₂₄ Alky-Morpholino, -C₁-C₂₄ Alky-Piperidino, -C₁-C₂₄ Alky-Piperazino, -C₁-C₂₄ Alky-Piperazino-N-Alkyl , Oxogruppen ( =O ) mit der Maßgabe, dass höchstens eine der Gruppen R¹, R² und R³ ein H-Atom darstellt,
sowie medizinische oder kosmetische topische Zubereitungen, enthaltend eine oder mehrere solcher Verbindungen in einer wirksamen Konzentration,
sowie die kosmetische Verwendung einer oder mehrerer solcher Verbindungen zur Verringerung der Sebumproduktion in der menschlichen Haut,
sowie die Verwendung von topischen Zubereitungen enthaltend einer oder mehrerer solcher Verbindungen zur Behandlung von fettiger und/oder unreiner Haut und/oder milden Formen der Akne und/oder fettigem Haar und/oder fettiger Kopfhaut und/oder der Seborrhö,
den Nachteilen des Standes der Technik abhelfen.

Die erfindungsgemäßen Verwendungen folgen einem gleichartigen Prinzip, da die Talgdrüsen auf der Kopfhaut und im Gesicht identisch aufgebaut sind und die Sebumproduktion nach demselben Mechanismus abläuft. So kann durch die Anwendung der sebumreduzierenden Wirkstoffe auf der Kopfhautregion die Sebumproduktion gesenkt und somit der kosmetisch unerwünschte Zustand der fettigen Haare verhindert werden.

Erfindungsgemäß sind solche Zubereitungen besonders vorteilhaft, die dadurch gekennzeichnet sind, dass eine oder mehrere Verbindungen der allgemeinen Formel (1), vorteilhaft in einer Wasser- und/oder Ölphase, in Konzentrationen von 0,0001 - 40,00 Gew.-%, bevorzugt 0,005 - 20,00 Gew.-%, besonders bevorzugt 0,001 - 6,00 Gew.-%, jeweils bezogen auf das Gesamtgewicht der Zusammensetzung, vorliegen.

Der Schutzumfang der Erfindung wird ausschließlich durch die Patentansprüche definiert. Weitere in der Beschreibung aufgeführte Ausführungsformen dienen lediglich zu Veranschaulichungszwecken. Bevorzugte Verbindungen der Formel (1) sind im Folgenden dargestellt wobei lediglich die Verbindungen T117e und T107e Verbindungen dieser Erfindung sind:

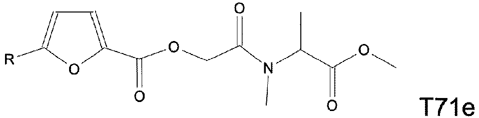

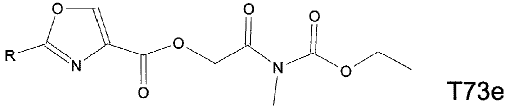

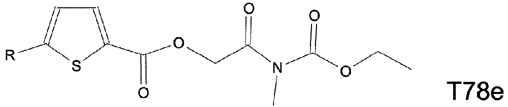

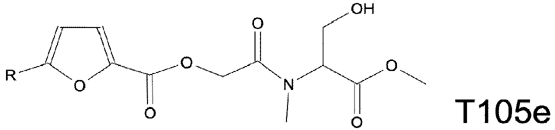

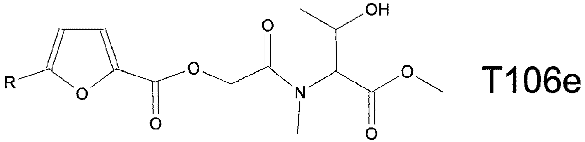

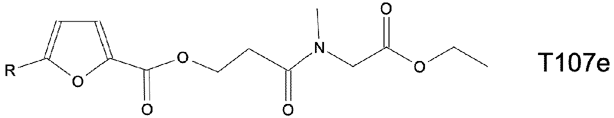

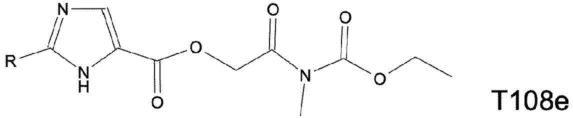

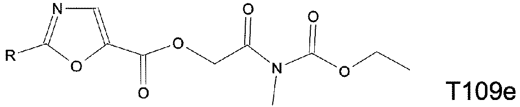

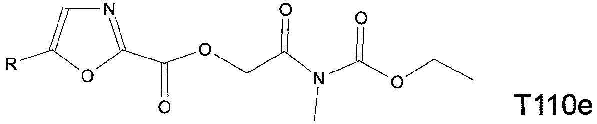

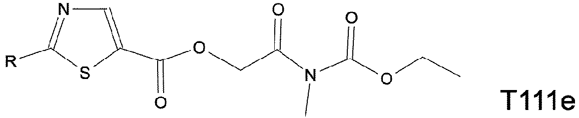

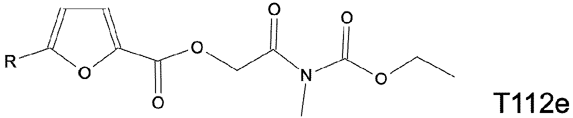

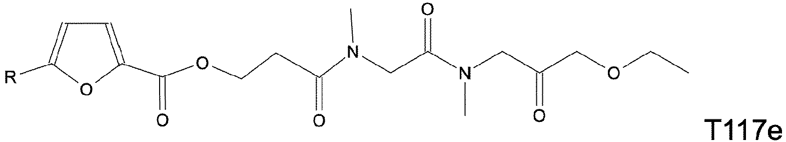

### Synthese von TOFA (3)

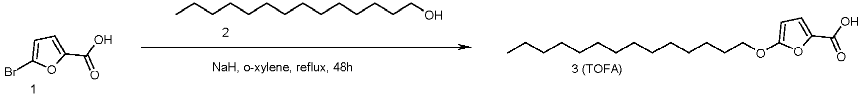

### Verbindung 3 (TOFA)

Zu einer Suspension von Alkohol (2) (25,3 g, 117,8 mM) in 400 ml o-Xylol wurde NaH (60 % in Mineralöl, 7,85 g, 196,3 mM) bei Umgebungstemperatur zugegeben und die Mischung wurde 2 Stunden lang unter Rückflusskühlung gerührt. Die Mischung wurde auf ca. 70°C herunter gekühlt, und Säure (1) wurde als Feststoff in einer Portion (15,0 g, 78,5 mM) zugegeben. Die Mischung wurde 48 h unter Rückflusskühlung gerührt und auf Umgebungstemperatur abgekühlt. Die Mischung wurde mit wässrigem KHSO₄ gequencht, mit EtOAc extrahiert, die organische Schicht wurde zusätzlich mit H₂O gewaschen, mit Na₂SO₄ getrocknet und eingedampft. Der Rückstand wurde mit Hexan verrieben, was Verbindung (3) (7,1 g, 28 %) ergab. Die Synthese wurde mehrmals wiederholt, um die erforderliche Menge an TOFA (ca. 100 g) zu erhalten.

### ZE33-0100 (BDF: T71e)

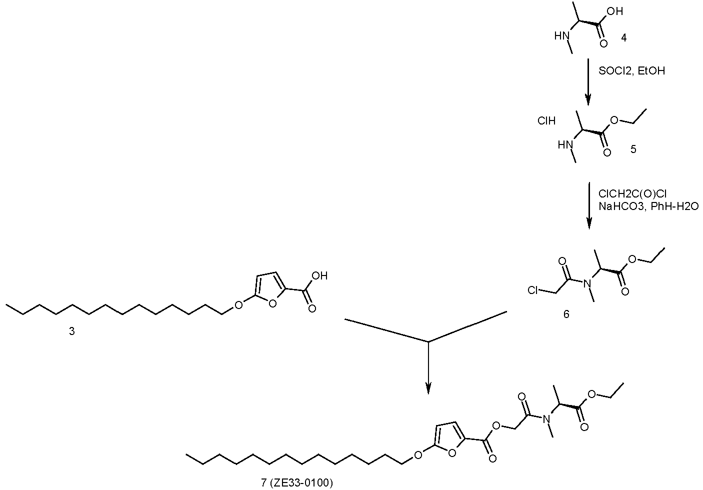

### Verbindung (5)

Zu einer Lösung von Verbindung (4) (15,0 g, 150 mM) in EtOH (100 ml) wurde bei 0 °C tropfenweise SOCl₂ (34,6 g, 300 mM) gegeben. Die resultierende Mischung wurde 12 h unter Rückflusskühlung erhitzt. Anschließend wurde die Mischung stehen gelassen, bis sie auf Raumtemperatur abkühlt war und dann im Vakuum eingeengt. Nach Entfernung des Lösungsmittels wurde der Rückstand in wässriges NaHCO₃ gegossen. Die wässrige Lösung wurde mit DCM extrahiert, und der Extrakt wurde nacheinander mit wässrigem NaHCO₃, H₂O, Kochsalzlösung gewaschen und über Na₂SO₄ getrocknet. Nach Aufkonzentration unter reduziertem Druck ergibt Verbindung (5) (18,0 g, 94%). Das Material wird im nächsten Schritt ohne Reinigung verwendet.

### Verbindung (6)

Verbindung (5) (12,4 g, 150 mM) wurde in 160 ml Wasser gelöst. Die Lösung wurde im Eisbad gekühlt und mit NaHCO₃ (14,3 g, 170,2 mM) in mehreren Schritten versetzt. Das Reaktionsgemisch wurde tropfenweise mit einer Lösung (7,5 g, 66,6 mM) von Chloracetylchlorid in 140 ml Toluol vermischt, dann 3 h bei 20-25°C kräftig gerührt. Nach einer Phasentrennung wurde die wässrige Phase mit Toluol extrahiert. Die vereinigten organischen Phasen wurden vom Lösungsmittel befreit, Verbindung (6) wurde als farbloses Öl erhalten (8,46 g, 55%).

### Verbindung (7) (ZE33-0100) (BDF: T71e)

Zu einer Lösung von TOFA (3) (15,4 g, 47,0 mM) in DMF (150 ml) wurde bei Raumtemperatur Verbindung (6) (1,2 Äq., 11,0 g, 56,0 mM) und Triethylamin (2 Äq., 9,5 ml, 94,0 l) gegeben mM). Nach der Zugabe wurde das Gemisch 18 h bei 50°C gerührt und dann wurde das Gemisch mit Ether (250 ml) verdünnt und mit Wasser (2 × 50 ml) gewaschen. Die zwei Schichten wurden getrennt und die wässrige Phase wurde mit Dichlormethan (2 × 50 ml) extrahiert. Die organischen Schichten wurden vereinigt, über wasserfreiem MgSO₄ getrocknet und unter reduziertem Druck eingedampft, um ein orangefarbenes Öl zu ergeben. Das resultierende Material wurde durch Silicagel-Säulenchromatographie (unter Elution mit 10-20% EtOAc/DCM) gereinigt, um Verbindung (7) (ZE33-0100) als weiße Kristalle (9,0 g, 39 %) herzustellen.

### ZE33-0101 (BDF: T105e)

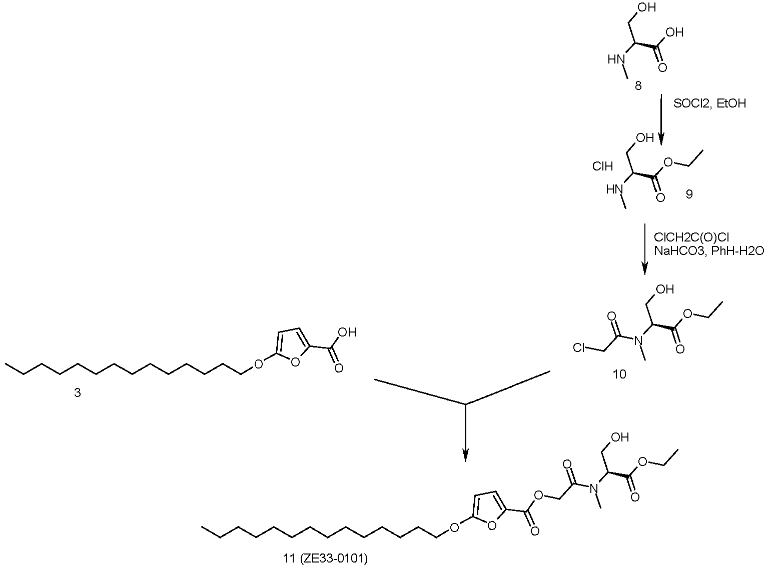

### Verbindung (9)

Zu einer Lösung von Verbindung (8) (2,0 g, 12,9 mM) in EtOH (20 ml) bei 0°C wurde tropfenweise SOCl₂; (7,6 g, 64,5 mM) zugegeben. Die resultierende Mischung wurde 12 h unter Rückflusskühlung erhitzt. Anschließend wurde die Mischung zum Abkühlen bei Raumtemperatur stehen gelassen und dann im Vakuum eingeengt. Nach Entfernung des Lösungsmittels wurde der Rückstand in wässriges NaHCO₃ gegossen. Die wässrige Lösung wurde mit DCM extrahiert, und der Extrakt wurde nacheinander mit wässrigem NaHCO₃, H₂O, Kochsalzlösung gewaschen und über Na₂SO₄ getrocknet. Aufkonzentration unter vermindertem Druck ergibt Verbindung (9) (2,2 g, 93%) als farbloses Pulver, das direkt für die nächste Reaktion verwendet wurde.

### Verbindung (10)

Verbindung (9) (2,2 g, 12,5 mM) wurde in 40 ml Wasser gelöst. Die Lösung wurde mittels eines Eisbades gekühlt und mit (2,6 g, 31,3 mM) NaHCO₃ in mehreren Schritten versetzt. Das Reaktionsgemisch wurde tropfenweise mit einer Lösung (1,3 g, 11,9 mM) von Chloracetylchlorid in 25 ml Toluol versetzt, dann 3 h bei 20-25°C kräftig gerührt. Nach einer Phasentrennung wurde die wässrige Phase mit Toluol extrahiert. Die vereinigten organischen Phasen wurden vom Lösungsmittel befreit, Verbindung (10) wurde als farbloses Öl (2,5 g, 90%) erhalten.

### Verbindung (11) (ZE33-0101) (BDF: T105e)

Zu einer Lösung von Verbindung (10) (10,0 g, 30,8 mM) in DMF (150 ml) wurde bei Raumtemperatur 4 (1,1 Äq., 7,6 g, 33,9 mM) und Triethylamin (2 Äq., 6,3 ml, 62,6 mM) zugegeben. Nach der Zugabe wurde das Gemisch 18 h bei 50 °C gerührt und dann wurde das Gemisch mit Ether (300 ml) verdünnt und mit Wasser (3 × 50 ml) gewaschen. Die zwei Schichten wurden getrennt und die wässrige Phase wurde mit Dichlormethan (2 × 50 ml) extrahiert. Die organischen Schichten wurden vereinigt, über wasserfreiem MgSO₄ getrocknet und unter reduziertem Druck eingedampft, um ein oranges Öl zu ergeben. Das resultierende Material wurde durch Silicagel-Säulenchromatographie (unter Elution mit 10-20% EtOAc/DCM) gereinigt, um Verbindung (11) (ZE33-0101) als weiße Kristalle (10,0 g, 63,4%) zu erhalten.

### ZE33-0102 (BDF: T106e)

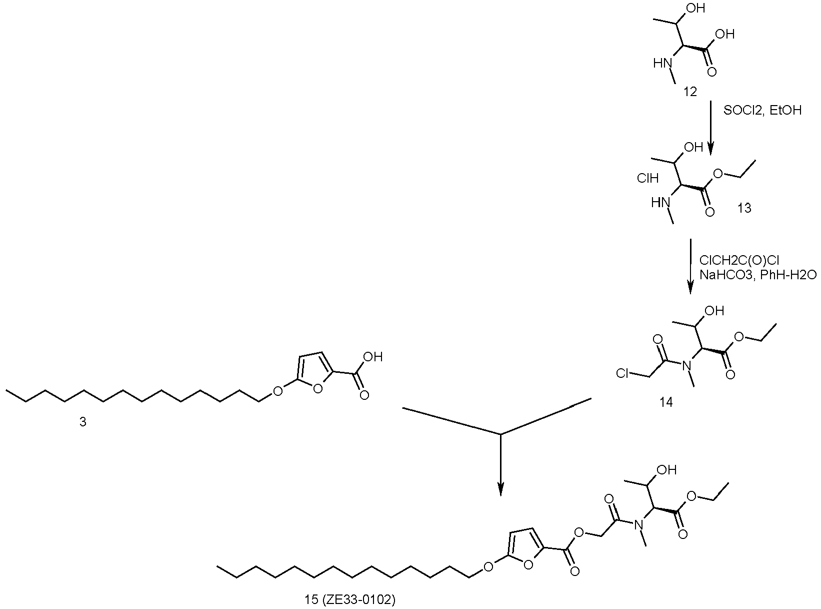

### Verbindung (13)

Zu einer Lösung von Verbindung (12) (14,0 g, 105,0 mM) in EtOH (200 ml) wurde bei 0°C tropfenweise SOCl₂ (16,3 g, 136,5 mM) zugegeben. Die resultierende Mischung wurde 1 h unter Rückflusskühlung erhitzt. Anschließend wurde die Mischung zum Abkühlen bei Raumtemperatur stehen gelassen und dann im Vakuum eingeengt. Nach Entfernen des Lösungsmittels wurde der Rückstand in wässriges NaHCO₃ gegossen. Die wässrige Lösung wurde mit DCM extrahiert, und der Extrakt wurde nacheinander mit wässrigem NaHCO₃, H₂O, Kochsalzlösung gewaschen und über Na₂SO₄ getrocknet. Aufkonzentration unter vermindertem Druck ergibt Verbindung (13) (20,5 g, 98%) als farbloses Pulver, das direkt für die nächste Reaktion verwendet wurde.

### Verbindung (14)

Verbindung (13) (20,5 g, 103,7 mM) wurde in 400 ml Wasser gelöst. Die Lösung wurde mittels Eisbad gekühlt und in mehreren Schritten mit (17,4 g, 207,4 mM) NaHCO₃ versetzt. Das Reaktionsgemisch wurde tropfenweise mit einer Lösung (12,9 g, 114,0 mM) Chloracetylchlorid in 150 ml Toluol versetzt, dann 3 h bei 20-25°C kräftig gerührt. Nach Phasentrennung wurde die wässrige Phase mit Toluol extrahiert. Die vereinigten organischen Phasen wurden vom Lösungsmittel befreit, um Verbindung (14) wurde als farbloses Öl (20,9 g, 85 %) zu erhalten.

### Verbindung 15 (ZE33-0102) (BDF: T106e)

Zu einer Lösung von TOFA (3) (12,0 g, 36,9 mM) in DMF (200 ml) wurde bei Raumtemperatur Verbindung (14) (1,2 Äq., 10,5 g, 44,3 mM) und Triethylamin (2 Äq., 7,5 ml, 73,8 mM) gegeben mM). Nach der Zugabe wurde das Gemisch 18 h bei 50 °C gerührt und dann wurde das Gemisch mit Ether (300 ml) verdünnt und mit Wasser (3 × 50 ml) gewaschen. Die zwei Schichten wurden getrennt und die wässrige Phase wurde mit Dichlormethan (2 × 50 ml) extrahiert. Die organischen Schichten wurden vereinigt, über wasserfreiem MgSO₄ getrocknet und unter reduziertem Druck eingedampft, um ein oranges Öl zu ergeben. Das resultierende Material wurde durch Silicagel-Säulenchromatographie (unter Elution mit 10-20% EtOAc/DCM) gereinigt, um Verbindung (15) (ZE33-0102) als weiße Kristalle (8,4 g, 43,2%) zu erhalten.

### ZE33-0104 (BDF: T107e) und ZE33-0147 (T117e)

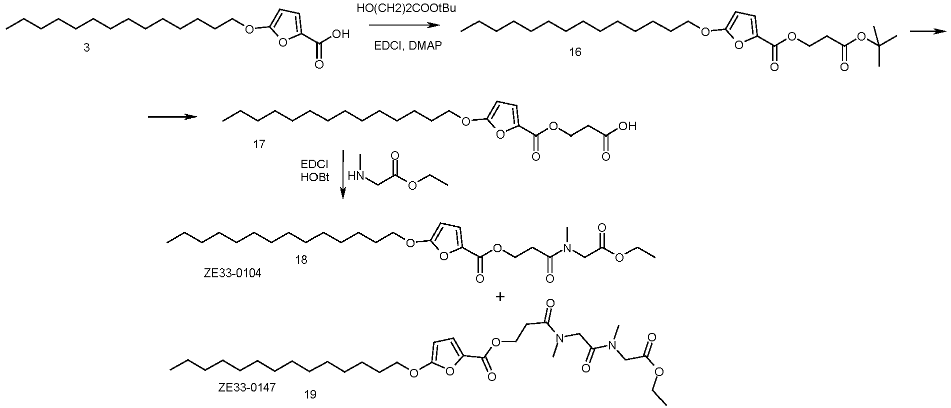

### Verbindung (16)

Zu einer Lösung von TOFA (3) (14,3 g, 44 mM) in 450 ml CH₂Cl₂ wurde HOCH₂CH₂COOt-Bu (9,1 g, 62 mM) gegeben, gefolgt von der Zugabe von EDCI (14,9 g, 77 mM) und DMAP (1,0 g, 8,2 mM). Die Mischung wurde 20 h bei Umgebungstemperatur gerührt, eingedampft, erneut in Et₂O gelöst, mit H₂O gewaschen und mit Na₂SO₄ getrocknet. Der Rückstand wurde durch Säulenchromatographie an Kieselgel gereinigt, wobei mit 5% EtOAc in Hexan eluiert wurde, was Verbindung (16) (12,5 g, 62 %) ergab.

### Verbindung (17)

Zu einer Lösung von Verbindung (16) (11,8 g, 26 mM) in 150 ml CH₂Cl₂ wurden 50 ml TFA gegeben und das Gemisch wurde 1 Stunde bei Umgebungstemperatur gerührt. Die Lösungsmittel wurden verdampft und der Rückstand wurde zusammen mit CCI 4 verdampft, was Verbindung (17) (10,5 g, 100 %) ergab.

### Verbindungen (18) and (19) (ZE33-0104 und ZE33-0147).

Zu einer Lösung von Verbindung (17) (11,1 g, 28 mM) in 500 ml CH₂Cl₂ wurde Sarcosinethylester-Hydrochlorid (5,6 g, 36,5 mM) gegeben, gefolgt von der Zugabe von HOBt (4,2 g, 31 mM), DIPEA (11,6 g, 90 mM) und EDCI (7,6 g, 40 mM). Die Mischung wurde 20 h bei Umgebungstemperatur gerührt, eingedampft, in EtOAc erneut gelöst, mit H₂O gewaschen und mit Na₂SO₄ getrocknet. Der Rückstand wurde durch Säulenchromatographie auf Silicagel gereinigt, wobei mit 0-50% EtOAc in Hexan eluiert wurde, um Verbindung (18) (ZE33-0104) (7,7 g, 56%) und Verbindung (19) (ZE33-0147) (3,19 g, 20%) herzustellen.

### ZE33-0105 (BDF: T72e)

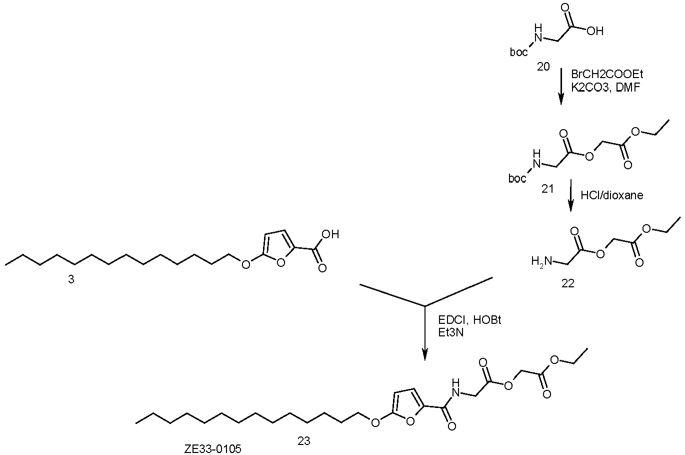

### Verbindung (21)

Zu einer Lösung von Boc-Glycin (20) (8,7 g, 50 mM) in 100 ml DMF wurde Ethylbromacetat (8,5 g, 50 mM) zugegeben, gefolgt von der Zugabe von K₂CO₃ (20,7 g, 150 mM). Die Mischung wurde 15 h bei 50-60 °C gerührt, auf Umgebungstemperatur abgekühlt, mit H₂O (400 ml) verdünnt und mit Et₂O extrahiert. Die vereinigten organischen Extrakte wurden mit Na₂SO₄ getrocknet und eingedampft, was Verbindung (21) (13,0 g, 99%) ergab, die für den nächsten Schritt ohne zusätzliche Reinigung verwendet wurde.

### Verbindung (22)

Zu einer Lösung von Verbindung (21) (13,0 g, 5 mM) in 100 ml CH₂Cl₂ wurden 25 ml TFA gegeben und die Mischung wurde bei Umgebungstemperatur 15 Stunden lang gerührt. Die flüchtigen Bestandteile wurden abgedampft und der Rückstand wurde mit wässrigem NaHCO₃ basisch gemacht. Die Lösung wurde mit CH₂Cl₂ extrahiert, die vereinigten organischen Extrakte wurden mit Na₂SO₄ getrocknet und eingedampft, was Verbindung (22) (8,0 g, 98%) ergab, die für den nächsten Schritt ohne zusätzliche Reinigung verwendet wurde.

### Verbindung (23) (ZE33-0105)

Zu einer Lösung von Verbindung (3) (TOFA) (5,3 g, 16 mM) und Amin (22) (3,9 g, 24 mM) in 200 ml CH₂Cl₂ wurde EDCI (6,3 g, 32 mM) gegeben, gefolgt von der Zugabe von DMAP (4,4 g, 32 mM) und das Gemisch wurde 24 h bei Umgebungstemperatur gerührt. Das Gemisch wurde mit wässriger Zitronensäure gequencht, die organische Schicht wurde mit wässrigem NaHCO 3 gewaschen, mit Na₂SO₄ getrocknet und eingedampft, was Verbindung (23) (ZE33-0105) (6,6 g, 88 %) ergab.

### ZE33-0107 (BDF: T108e) und ZE33-0119 (T114s)

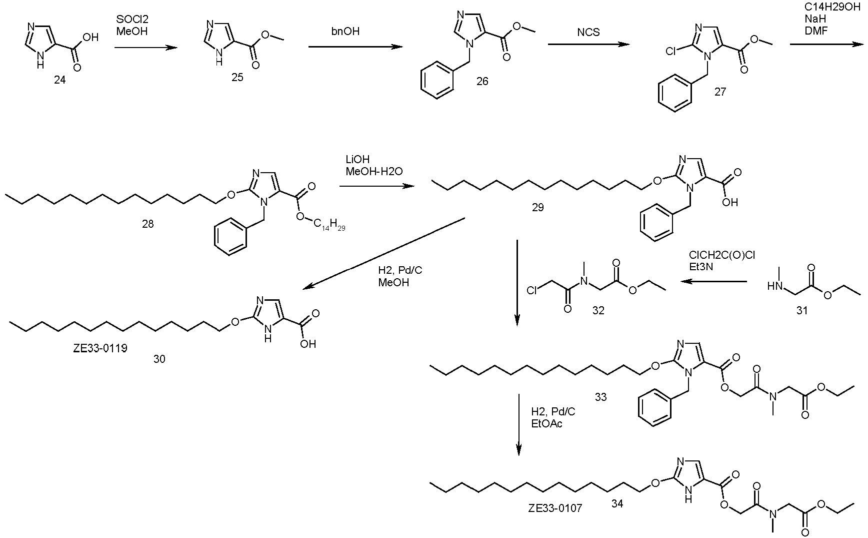

### Verbindung (25)

Zu einer Suspension von Imidazol-2-carbonsäure (24) (51,4 g, 459 mM) in 400 ml MeOH wurde SOCl₂ tropfenweise (109,1 g, 918 mM) zugegeben. Die Mischung wurde 15 h unter Rückflusskühlung gerührt, auf Umgebungstemperatur abgekühlt und eingedampft. Der Rückstand wurde mit wässrigem K₂CO₃ basisch gemacht, mit t-BuOH-CHCl₃, 1:1 extrahiert. Die vereinigten Extrakte wurden mit Na₂SO₄ getrocknet, eingedampft und zweimal mit MeCN zusammen eingedampft, was Verbindung (25) (51,0 g, 88 %) ergab.

### Verbindung (26)

Diisolpopylazadicarboxylat (98,1 g, 485 mM) in 100 ml THF wurde tropfenweise zu einer Mischung von Verbindung (25) (51,0 g, 404 mM), Benzylalkohol (52,4 g, 485 mM) und PPh3 (127,3 g, 485 mM) in 500 ml THF bei 5-10°C gegeben. Das Gemisch wurde 15 h bei Umgebungstemperatur gerührt und anschließend eingedampft. Der Rückstand wurde in EtOAc gelöst und die Zielverbindung wurde mit 10 % wässriger HCl extrahiert. Die vereinigten wässrigen Extrakte wurden vorsichtig mit festem K₂CO₃ bei 5-10 °C basisch gemacht. Die Mischung wurde mit EtOAc extrahiert, die vereinigten organischen Extrakte wurden mit Na₂SO₄ getrocknet und eingedampft. Der Rückstand wurde durch Säulenchromatographie auf Silicagel gereinigt, wobei mit 25-50% EtOAc in Hexan eluiert wurde, was Verbindung (26) (68,1 g, 78%) ergab.

### Verbindung (27)

Eine Mischung von Verbindung (26) (68,1 g, 315 mM) und N-Chlorsuccinimid (42,0 g, 315 mM) in 350 ml DMF wurde 15 Stunden bei Umgebungstemperatur gerührt. Die Reaktionsmischung wurde mit wässrigem NaHCO₃ gequencht, mit Et₂O extrahiert, die vereinigten organischen Extrakte wurden mit Na₂SO₄ getrocknet und eingedampft. Der Rückstand wurde durch Säulenchromatographie an Kieselgel gereinigt, wobei mit 1-10% EtOAc in Hexan eluiert wurde. Die Zielfraktionen wurden eingedampft und der feste Rückstand wurde mit Hexan gewaschen, was Verbindung (27) (32,8 g, 42 %) ergab.

### Verbindung (28)

Zu einer Suspension von n-Tetradecanol (70,1 g, 328 mM) in 600 ml DMF wurde portionsweise NaH (60% in Mineralöl, 13,1 g, 328 mM) zugegeben. Das Gemisch wurde 2 h bei 50 °C gerührt und Verbindung (27) (32,8 g, 131 mM) wurde als Feststoff zugegeben. Das Reaktionsgemisch wurde 24 h bei 50 °C und dann ein Wochenende lang bei Umgebungstemperatur gerührt. Die Mischung wurde mit wässrigem NH4Cl gequencht, mit EtOAc extrahiert, die vereinigten organischen Extrakte wurden mit Na₂SO₄ getrocknet und eingedampft. Der Rückstand wurde durch Säulenchromatographie an Kieselgel gereinigt, wobei mit 1-5% EtOAc in Hexan eluiert wurde. Die Zielfraktionen wurden eingedampft und der feste Rückstand wurde mit MeOH gewaschen, was Verbindung (28) (17,8 g, 22 %) ergab.

### Verbindung (29)

Eine Mischung aus Verbindung (28) (17,8 g, 29 mM) und LiOH·H₂O (9,8 g, 232 mM) in MeOH-THF-H₂O (300 ml) wurde 15 h bei 80 °C und dann 24 h bei Umgebungstemperatur gerührt. Die organischen Lösungsmittel wurden verdampft und der wässrige Rückstand wurde mit wässrigem KHSO₄ angesäuert. Die Mischung wurde mit EtOAc extrahiert, die vereinigten organischen Extrakte wurden mit Na₂SO₄ getrocknet und eingedampft. Der feste Rückstand wurde mit Hexan gewaschen, was Verbindung (29) (11,3 g, 93%) ergab.

### Verbindung (30) (ZE33-0119).

Zu einer Lösung von Verbindung (29) (0,31 g, 0,75 mM) in MeOH wurden zuerst einige Tropfen konzentrierte HCl zugegeben und anschließend 10 % Pd/C (30 mg). Die Mischung wurde bei 7 MPa 2 Stunden lang bei 40°C hydriert. Der Katalysator wurde abfiltriert und das Lösungsmittel wurde verdampft. Der Rückstand wurde erneut in MeOH gelöst und mit Dowex 1X8 Chloridform (stark basisch) basisch gemacht. Die Mischung wurde durch Celite abfiltriert und eingedampft. Der Rückstand wurde in heißem MeCN suspendiert, abfiltriert und getrocknet, was Verbindung (30) (ZE33-0119) (0,16 g, 67%) ergab.

### Verbindung (32)

Sarcosinethylester-Hydrochlorid (31) (50,00 g, 330 mM) wurde in 160 ml Wasser gelöst. Die Lösung wurde mittels Eisbades gekühlt und in mehreren Schritten mit 2,5 eq (68,4 g, 830 mM) NaHCO 3 versetzt. Das Reaktionsgemisch wurde tropfenweise mit einer Lösung von (36,7 g, 330 mM) Chloracetylchlorid in 140 ml Toluol vermischt, dann 3 h bei 20-25°C kräftig gerührt. Nach Phasentrennung wurde die wässrige Phase mit Toluol extrahiert. Die vereinigten organischen Phasen wurden vom Lösungsmittel befreit. (53,5 g, 84,9%) der Verbindung (32) wurde als farbloses Öl erhalten.

### Verbindung (33)

Eine Mischung aus Verbindung (29) (11,3 g, 27 mM), Chloracetylchlorid (32) (6,9 g, 35 mM) und Et3N (16,5 g, 162 mM) in 200 ml DMF wurde 15 h bei 60°C gerührt. Die Mischung wurde mit wässrigem NH4Cl gequencht, mit EtOAc extrahiert, die vereinigten organischen Extrakte wurden mit Na₂SO₄ getrocknet und eingedampft. Der Rückstand wurde durch Säulenchromatographie an Kieselgel gereinigt, wobei mit 20-75% EtOAc in Hexan eluiert wurde. Die Zielfraktionen wurden eingedampft und der feste Rückstand wurde mit Hexan

### Verbindung (34) (ZE33-0107)

Zu einer Lösung von Verbindung (33) (11,6 g, 20,3 mM) in 150 ml EtOAc wurden 2,32 g 10% Pd/C zugegeben und die Mischung wurde bei 5 MPa und 40-50 °C für 48 Stunden hydriert. Der Katalysator wurde abfiltriert, das Filtrat wurde eingedampft und der Rückstand wurde durch Säulenchromatographie an Kieselgel gereinigt, wobei mit 20-50% EtOAc in Hexan 25% CHCl3 in EtOAc eluiert wurde, was Verbindung (34) (ZE33-0107) (5,82 g, 60%) ergibt.

### ZE33-0110 (T109e) und ZE33-0122

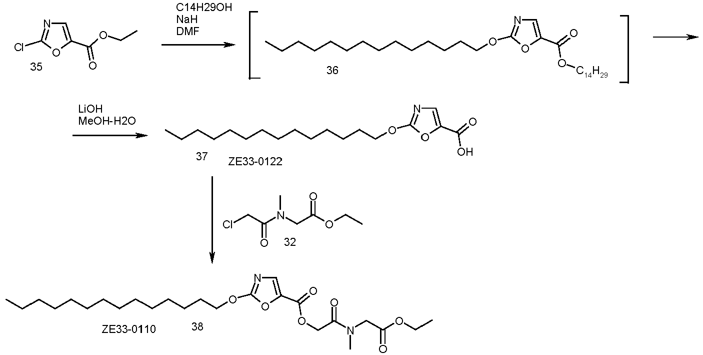

### Verbindung (37) (ZE33-0122)

Zu einer Lösung von n-Tetradecanol (12,3 g, 57,5 mM) in 100 ml THF wurde 60 % NaH (1,82 g, 46 mM) zugegeben. Das Gemisch wurde 4 h bei 80 °C gerührt, auf 5 °C abgekühlt und eine Lösung von Chloroxazol (35) (6,08 g, 28,9 mM) in 20 ml THF wurde zugegeben. Die Mischung wurde 15 h bei Umgebungstemperatur gerührt und mit 5% HCl gequencht. Die Mischung wurde mit Et₂O extrahiert, die vereinigten organischen Extrakte wurden mit Na₂SO₄ getrocknet und eingedampft. Der Rückstand wurde mit Hexan gewaschen und getrocknet, wodurch Verbindung (36) (4,4 g) erhalten wurde, die ohne zusätzliche Reinigung für den nächsten Schritt verwendet wurde. Verbindung (36) wurde in 250 ml THF und 75 ml MeOH wieder gelöst, 1,55 g LiOH und 5 ml H₂O wurden zugegeben und das Gemisch wurde 15 h bei Umgebungstemperatur gerührt. Die organischen Bestandteile wurden verdampft und der Rückstand wurde mit 1,5% HCl gequencht. Das gebildete Pellet wurde mit H₂O gewaschen und getrocknet, was Verbindung (37) (ZE33-0122) (2,04 g, 22 %) ergab.

### Verbindung (38) (ZE33-0110)

Eine Mischung von Verbindung (37) (6,1 g, 187 mM), Chlorid (32) (9,0 g, 46 mM) und Et3N (9,0 g, 89 mM) in 200 ml MTBE wurde 48 h bei 60°C gerührt. Die Mischung wurde mit wässrigem NH4Cl gequencht, mit EtOAc extrahiert, die vereinigten organischen Extrakte wurden mit Na₂SO₄ getrocknet und eingedampft. Der Rückstand wurde durch Säulenchromatographie an Kieselgel gereinigt, wobei mit 20-50% EtOAc in Hexan eluiert wurde. Die Zielfraktionen wurden eingedampft und der feste Rückstand wurde mit 10 % Et₂O in Hexan gewaschen, was Verbindung (38) (ZE33-0110) (5,0 g, 55 %) ergab.

### ZE33-0111 (T110e)

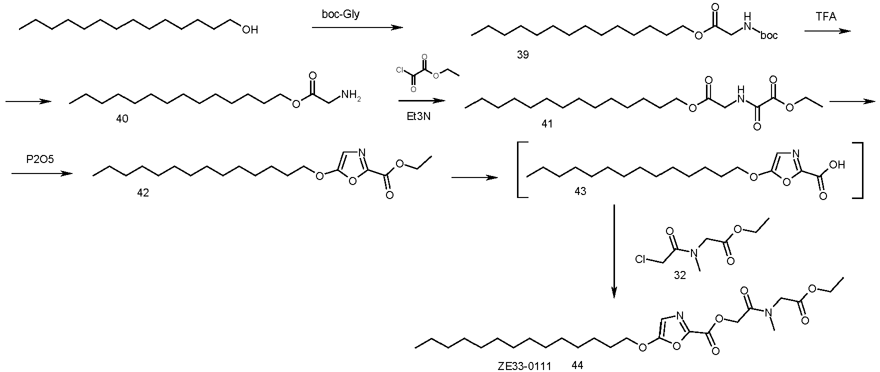

### Verbindung (39)

Zu einer Lösung von n-Tetradecanol (20,0 g, 92 mM) in 800 ml CH₂Cl₂ wurde EDCI (21,6 g, 112 mM) zugegeben, gefolgt von der Zugabe von DMAP (1,12 g, 0,2 mM) und die Mischung wurde bei Umgebungstemperatur für 24 Stunden gerührt. Die Mischung wurde mit 20% wässriger H₂SO₄ gequencht, die organische Schicht wurde mit wässriger NaHCO₃ gewaschen, mit Na₂SO₄ getrocknet und eingedampft, was Verbindung (39) (34,8 g, 100%) ergab.

### Verbindung (40)

Zu einer Lösung von Verbindung (39) (34,8 g, 92 mM) in 200 ml CH₂Cl₂ wurden 50 ml TFA gegeben und die Mischung wurde bei Umgebungstemperatur 15 Stunden lang gerührt. Die flüchtigen Bestandteile wurden abgedampft und der Rückstand wurde mit wässrigem NaHCO₃ basisch gemacht. Die Lösung wurde mit CH₂Cl₂ extrahiert, die vereinigten organischen Extrakte wurden mit Na₂SO₄ getrocknet und eingedampft, was Verbindung (40) (20,0 g, 80%) ergab.

### Verbindung (41)

Zu einer Lösung von Verbindung (40) (20,0 g, 72 mM) in CH₂Cl₂ (300 ml) wurde Et₃N (22,4 g, 216 mM) zugegeben, gefolgt von der tropfenweisen Zugabe von Ethyloxalylchlorid (12,0 g, 88 mM) in 40 ml CH₂Cl₂ bei 5°C. Die Reaktionsmischung wurde 4 h bei Umgebungstemperatur gerührt, mit 10% HCl gequencht, mit CH₂Cl₂ extrahiert, die vereinigten organischen Extrakte wurden mit Na₂SO₄ getrocknet und eingedampft. Der Rückstand wurde durch Säulenchromatographie an Kieselgel gereinigt, wobei mit 5-30% EtOAc in Hexan eluiert wurde, was Verbindung (41) ergab (12,0 g, 43%).

### Verbindung (42)

Eine Suspension von P₂O₅ (84,4 g, 600 mM) in CH₃CN (150 ml) unter Stickstoff wurde auf 50 °C erwärmt und mit Verbindung (41) (36,8 g, 100 mM) gelöst in 30 ml CH₃CN behandelt. Die Mischung wurde 1,5 Stunden auf 65°C erhitzt, dann in einem Eisbad gekühlt. Eis und Kochsalzlösung wurden dem Reaktionsgemisch zugesetzt, dann wurde EtOAc zugegeben und das Gemisch in einen Scheidetrichter überführt. CHC1₃ wurde zugegeben, um Feststoffe aufzulösen, und die organische Schicht wurde isoliert. Die wässrige Schicht wurde wiederholt mit CHC1₃ und EtOAc gewaschen, die organischen Schichten wurden vereinigt und mit Na₂SO₄ getrocknet und dann aufkonzentriert. Der Rückstand wurde an Kieselgel chromatographiert, wobei mit einem Gradienten von 0-30% EtOAc/Hexan eluiert wurde, um das Produkt als farbloses Öl zu ergeben, das zusätzlich mit Hexan umkristallisiert wurde, was Verbindung (42) (27 g, 77%) ergab.

### Verbindung (43)

Ein Gemisch von Verbindung (42) (20,5 g, 60 mM) und LiOH (3,7 g, 90 mM) in einem Lösungsmittelgemisch THF/MeOH/H₂O (50 ml, 4 ml, 150 ml) wurde bei 50°C für 4 Stunden gerührt. Die Reaktionsmischung wurde mit wässriger 2N HCl auf pH 2 angesäuert und der resultierende Niederschlag wurde abfiltriert und zuerst mit H₂O, dann mit EtOAc gewaschen, um Verbindung (43) als weißen Feststoff bereitzustellen, der für den nächsten Schritt ohne zusätzliche Reinigung verwendet wurde (15,4 g, 82%).

### Verbindung (44) (ZE33-0111)

Zu einer Lösung von Verbindung 43 (15,4 g, 47 mM) in DMF (150 ml) wurde bei Raumtemperatur Verbindung (32) (11,0 g, 56 mM) und Triethylamin (9,5 ml, 94 mM) gegeben. Nach der Zugabe wurde das Gemisch 18 h bei 50 °C gerührt und dann wurde das Gemisch mit Ether (250 ml) verdünnt und mit Wasser (2 × 50 ml) gewaschen. Die zwei Schichten wurden getrennt und die wässrige Phase wurde mit Dichlormethan (2 × 50 ml) extrahiert. Die organischen Schichten wurden vereinigt, über wasserfreiem MgSO₄ getrocknet und unter reduziertem Druck eingedampft, um ein oranges Öl zu ergeben. Der Rückstand wurde durch Silicagel-Säulenchromatographie (unter Elution mit 10-20% EtOAc/DCM) gereinigt, um Verbindung (44) (ZE33-0111) (9,0 g, 39 %) bereitzustellen.

### ZE33-0112 (T111e) and ZE33-0124

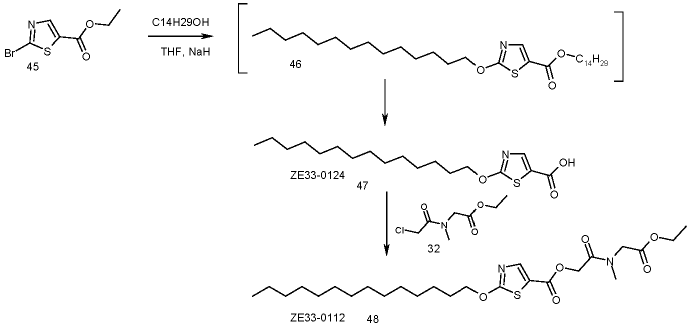

### Verbindung (47) (ZE33-0124)

Zu einer Suspension von n-Tetradecanol (41,7 g, 195 mM) in 200 ml DMF wurde NaH (60% in Mineralöl) portionsweise (7,4 g, 185 mM) zugegeben. Die Mischung wurde 2 h bei 60 °C gerührt und Verbindung (45) (23,0 g, 97 mM) wurde in 50 ml DMF zugegeben. Das Reaktionsgemisch wurde 24 h bei 80 °C gerührt. Die Mischung wurde mit 10 % wässriger H₂SO₄ gequencht, mit EtOAc extrahiert, die vereinigten organischen Extrakte wurden mit Na₂SO₄ getrocknet und eingedampft. Der Rückstand wurde mit 10 % MTBE in Hexan gewaschen, was Verbindung (47) (ZE33-0124) (7,4 g, 22 %) ergab.

### Verbindung (48) (ZE33-0112)

Eine Mischung von Verbindung (47) (7,4 g, 22 mM), Chlorid (32) (4,6 g, 24 mM) und Et₃N (6,6 g, 66 mM) in 200 ml MTBE wurde 48 h bei 60°C gerührt. Die Mischung wurde mit wässrigem NH₄Cl gequencht, mit EtOAc extrahiert, die vereinigten organischen Extrakte wurden mit Na₂SO₄ getrocknet und eingedampft. Der Rückstand wurde mit MeCN-H₂O, 1:1 gewaschen, gefolgt von Waschen mit Et₂O und Trocknen, was Verbindung (48) (8,8 g, 82 %) ergab.

### ZE33-0113 (T73e) and ZE33-0125

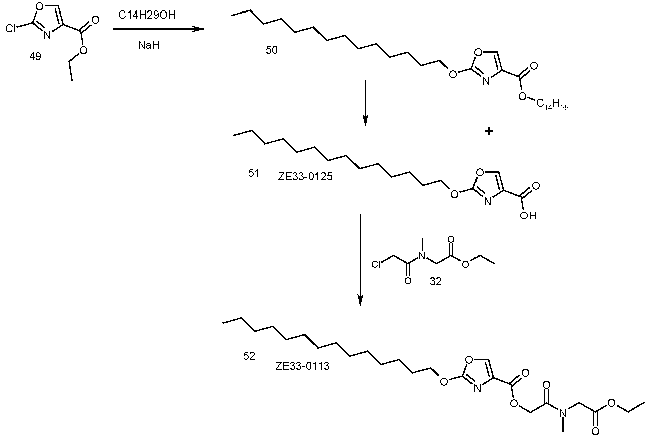

### Verbindung (50)

Zu einer Lösung von n-Tetradecanol (60,0 g, 280 mM) in 500 ml THF wurde 60 % NaH (8,0 g, 245 mM) zugegeben. Das Gemisch wurde 4 h bei 80 °C gerührt, auf 5 °C abgekühlt und eine Lösung von Chloroxazol (49) (19,5 g, 111 mM) in 50 ml THF wurde zugegeben. Die Mischung wurde 15 h bei Umgebungstemperatur gerührt und mit 5% HCl gequencht. Die Mischung wurde mit Et₂O extrahiert, die vereinigten organischen Extrakte wurden mit Na₂SO₄ getrocknet und eingedampft. Der Rückstand wurde durch Säulenchromatographie an Kieselgel (EtOAc-Hx, 0-75 %) gereinigt, was Verbindung (50) (20,0 g, 35 %) ergab.

### Verbindung (51) (ZE33-0125)

Verbindung (50) (19,6 g, 38 mM) wurde in 250 ml THF und 75 ml MeOH gelöst, 4,6 g (190 mM) LiOH und 20 ml H₂O wurden zugegeben und das Gemisch wurde 15 h bei Umgebungstemperatur gerührt. Die organischen Bestandteile wurden verdampft und der Rückstand wurde mit 1,5% HCl gequencht. Die Mischung wurde mit CH₂Cl₂ extrahiert, die vereinigten organischen Extrakte wurden mit Na₂SO₄ getrocknet und eingedampft. Der Rückstand wurde durch Säulenchromatographie an Kieselgel gereinigt, wobei mit 0-10% EtOAc in CH₂Cl₂ eluiert wurde, was Verbindung (51) (ZE33-0125) (19,6 g, 100%) ergab.

### Verbindung (52) (ZE33-0113)

Eine Mischung von Verbindung (51) (19,5 g, 38 mM), Chlorid (32) (11,0 g, 57 mM) und Et₃N (17,3 g, 170 mM) in 200 ml DMF wurde 48 h bei 60 °C gerührt. Die Mischung wurde mit wässrigem NH₄Cl gequencht, mit EtOAc extrahiert, die vereinigten organischen Extrakte wurden mit Na₂SO₄ getrocknet und eingedampft. Der Rückstand wurde durch Säulenchromatographie an Kieselgel gereinigt, wobei mit 20-50% EtOAc in Hexan eluiert wurde. Die Zielfraktionen wurden eingedampft und der feste Rückstand wurde mit 10% Et₂O in Hexan gewaschen, was Verbindung (52) (ZE33-0113) (8,77 g, 48%) ergab.

### ZE33-0114 (T78e) and ZE33-0126

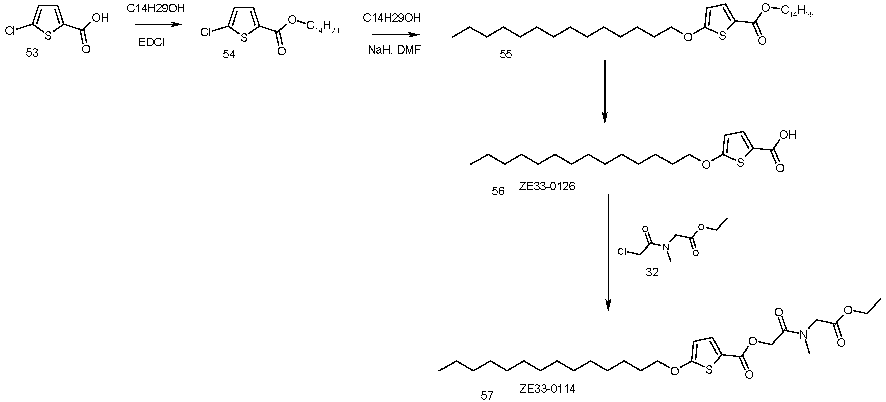

### Verbindung (54)

Zu einer Lösung von Chlorthiophencarbonsäure (53) (25 g, 154 mM) und n-Tetradecanol (39,5 g, 185 mM) in 400 ml CH₂Cl₂ wurde EDCI (35,4 g, 185 mM) gegeben, gefolgt von der Zugabe von DMAP (3,8 g .). , 31 mM) und das Gemisch wurde 5 h bei Umgebungstemperatur gerührt. Die Mischung wurde mit 20% wässriger H₂SO₄ gequencht, die organische Schicht wurde mit Na₂SO₄ getrocknet, durch eine SiO₂ -Schicht geleitet und eingedampft, was Verbindung (54) (52,8 g, 96%) ergab.

### Verbindung (55)

Zu einer Suspension von n-Tetradecanol (63,0 g, 294 mM) in 400 ml DMF wurde NaH (60% in Mineralöl) portionsweise (11,2 g, 279 mM) zugegeben. Die Mischung wurde 3 h bei 60 °C gerührt und Verbindung (54) (52,8 g, 147 mM) wurde in 100 ml DMF zugegeben. Das Reaktionsgemisch wurde 24 h bei 80 °C gerührt. Die Mischung wurde mit 10 % wässriger H₂SO₄ gequencht, mit EtOAc extrahiert, die vereinigten organischen Extrakte wurden mit Na₂SO₄ getrocknet und eingedampft. Der Rückstand wurde durch Säulenchromatographie an Kieselgel gereinigt, wobei mit 1-5% EtOAc in Hexan eluiert wurde. Die Zielfraktionen wurden eingedampft und der feste Rückstand wurde mit MeOH gewaschen, was Verbindung (55) (38,9 g, 49%) ergab.

### Verbindung (56) (ZE33-0126).

Zu einer Lösung von Verbindung (55) (38,9 g, 72 mM) in 1000 ml EtOH-H₂O, 1:1, wurden 50 ml 10 N wässrige NaOH zugegeben und das Gemisch wurde 15 h bei 120 °C gerührt. MeOH wurde verdampft, Et₂O wurde zugegeben und die mittlere Schicht, die die Zielverbindung enthielt, wurde abgetrennt und mit 10 % wässriger H₂SO₄ angesäuert. Die vereinigten Ether-Extrakte wurden mit Na₂SO₄ getrocknet und eingedampft, der feste Rückstand wurde mit Hexan gewaschen und getrocknet, was Verbindung (56) (ZE33-0126) (14,3 g, 58%) ergab.

### Verbindung (57) (ZE33-0114)

Eine Mischung von Verbindung (56) (7,2 g, 22 mM), Chlorid (32) (4,0 g, 22 mM) und Et₃N (4,2 g, 44 mM) in 200 ml DMF wurde 15 h bei 60°C gerührt. Die Mischung wurde mit wässrigem NH₄Cl gequencht, mit EtOAc extrahiert, die vereinigten organischen Extrakte wurden mit Na₂SO₄ getrocknet und eingedampft. Der Rückstand wurde durch Säulenchromatographie an Kieselgel gereinigt, wobei mit 20-30% EtOAc in Hexan eluiert wurde. Die Zielfraktionen wurden eingedampft und der feste Rückstand wurde mit 10% Et₂O in Hexan gewaschen, was Verbindung (57) (ZE33-0114) (7,38 g, 70%) ergab.

### ZE33-0115 (T112e) and ZE33-0127

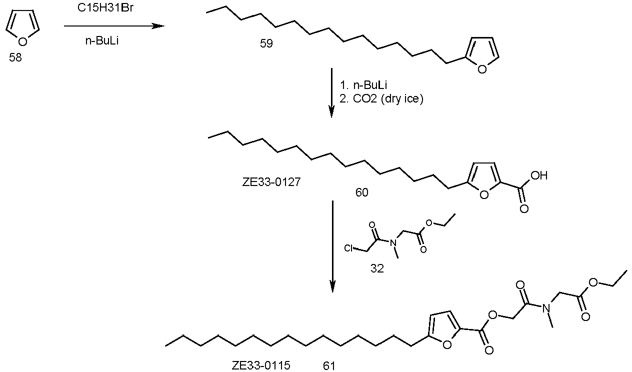

### Verbindung (59)

Zu einer Lösung von Furan (58) (4,9 g, 72 mM) in 30 ml THF wurden 30 ml 2,5 M n-BuLi in Hexan tropfenweise bei -70°C zugegeben. Das Gemisch wurde 0,5 h bei -70 °C und 1 h bei 0 °C gerührt, auf -70 °C abgekühlt und mit einer Lösung von n-Pentadecylbromid (18,0 g, 62 mM) in 20 ml THF versetzt. Die Mischung wurde 15 h bei Raumtemperatur gerührt, mit wässrigem NH₄Cl gequencht, mit Et₂O extrahiert, die vereinigten organischen Extrakte wurden mit Na₂SO₄ getrocknet und eingedampft. Der Rückstand wurde durch Säulenchromatographie an Kieselgel unter Elution mit Hexan gereinigt, was zu Verbindung (59) (11,9 g, 69 %) führte.

### Verbindung (60) (ZE33-0127)

Zu einer Lösung von Verbindung (59) (9,7 g, 35 mM) in 100 ml THF wurden 20 ml 2,5 M *n-*BuLi in Hexan tropfenweise bei -30°C zugegeben. Die Mischung wurde 3,5 h bei Umgebungstemperatur gerührt, auf -35°C abgekühlt und mit frisch zerstoßenem Trockeneis (-400 g) versetzt. Die Mischung wurde 15 h bei Umgebungstemperatur gerührt und mit wässrigem KHSO₄ gequencht. Die Mischung wurde mit CH₂Cl₂ extrahiert, die vereinigten organischen Extrakte wurden mit Na₂SO₄ getrocknet und eingedampft. Der feste Rückstand wurde mit Hexan gewaschen, was Verbindung (60) (ZE33-0127) (9,5 g, 91 %) ergab.

### Verbindung (61) (ZE33-0115)

Eine Mischung von Verbindung (60) (11,6 g, 36 mM), Chlorid (32) (11,7 g, 60 mM) und Et₃N (35 g, 35 mM) in 150 ml DMF wurde 24 h bei 60°C gerührt. Die Mischung wurde mit wässrigem NH₄Cl gequencht, mit EtOAc extrahiert, die vereinigten organischen Extrakte wurden mit Na₂SO₄ getrocknet und eingedampft. Der Rückstand wurde durch Säulenchromatographie auf Silicagel gereinigt, wobei mit 0 ± 5% EtOAc in CH₂Cl₂ eluiert wurde, was Verbindung (61) (ZE33-0115) (13,2 g, 76%) ergab.

### ZE33-0116 (T113e)

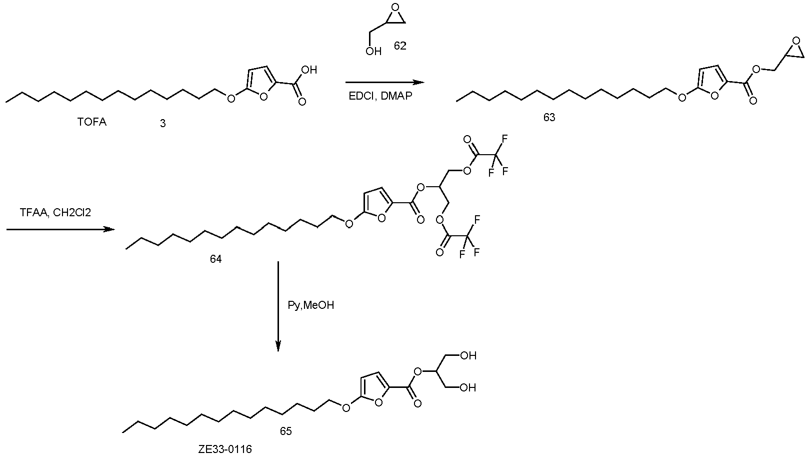

### Verbindung (63)

Zu einer Lösung von TOFA (3) (13,1 g, 40 mM) in 500 ml CH₂Cl₂ wurde Glycidol (62) (3,78 g, 51 mM) gegeben, gefolgt von der Zugabe von EDCI (10,5 g, 55 mM) und DMAP (0,97 g, 8 mM) und das Gemisch wurde 20 h bei Umgebungstemperatur gerührt. Das Lösungsmittel wurde verdampft, der Rückstand wurde erneut in EtOAc gelöst, mit H₂O gewaschen und über Na₂SO₄ getrocknet und eingedampft. Der Rückstand wurde durch Säulenchromatographie an Kieselgel gereinigt, wobei mit 15% EtOAc in Hexan eluiert wurde, was Verbindung (63) (9,43 g, 61%) ergab.

### Verbindung (64)

Zu einer Lösung von 9,43 g (25 mM) Verbindung (63) in 120 ml CH₂Cl₂ wurde Trifluoressigsäureanhydrid (26,4 g, 126 mM) in 40 ml CH₂Cl₂ bei -30°C gegeben. Das Gemisch wurde 4 Stunden bei Umgebungstemperatur gerührt und eingedampft, der Rückstand wurde zusammen mit CCl₄ eingedampft, was Verbindung (64) (14,5 g, 99%) ergab.

### Verbindung (65) (ZE33-0116)

Zu einer Lösung von Verbindung (64) (14,5 g, 25 mM) in 120 ml CH₂Cl₂ bei -55 °C wurde eine Lösung von Pyridin (22 g) und MeOH (13 g) in 100 ml CH₂Cl₂ tropfenweise zugegeben. Die Mischung wurde 0,5 h bei -30°C und dann 3 h bei Umgebungstemperatur gerührt. Die Mischung wurde eingedampft und der Rückstand wurde mit Hexan gewaschen, was Verbindung (65) (ZE33-0116) (9,85 g, 100%) ergab.

### ZE33-0117 (T74e)

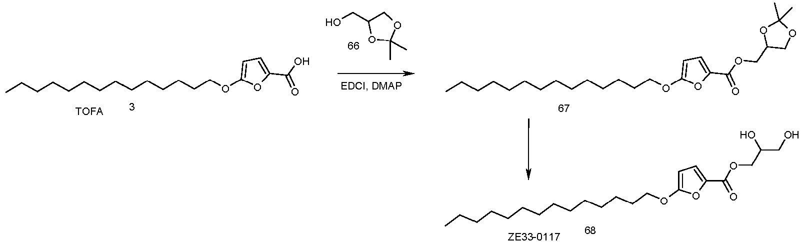

### Verbindung (67)

Zu einer Lösung von TOFA (3) (7,9 g, 24 mM) in 500 ml CH₂Cl₂ wurde geschütztes Glycerin (66) (4,1 g, 31 mM) zugegeben, gefolgt von der Zugabe von EDCI (6,3 g, 33 mM) und DMAP (0,61 ). g, 5 mM) und das Gemisch wurde 20 h bei Umgebungstemperatur gerührt. Das Lösungsmittel wurde verdampft, der Rückstand wurde erneut in EtOAc gelöst, mit H₂O gewaschen und über Na₂SO₄ getrocknet und eingedampft. Der Rückstand wurde durch Säulenchromatographie an Kieselgel gereinigt, wobei mit 15% EtOAc in Hexan eluiert wurde, was Verbindung (67) (9,16 g, 86%) ergab.

### Verbindung (68) (ZE33-0117)

Zu einer Lösung von Verbindung (67) (8,78 g, 20 mM) in 400 ml MeOH wurden 0,4 ml 3 M HCl in Dioxan zugegeben und das Gemisch wurde 0,5 Stunden bei Umgebungstemperatur gerührt. Die Mischung wurde eingedampft und anschließend zusammen mit MeOH auf ca. 20 ml Volumen eingedampft, das entstandene Pellet wurde abfiltriert und getrocknet, was Verbindung (68) (ZE33-0117) (6,9 g, 87%) ergab.

Die erfindungsgemäßen Verbindungen können vorteilhaft in kosmetische oder dermatologische Zubereitungen eingearbeitet werden.

Die erfindungsgemäßen Zubereitungen bzw. Verwendungen können in Form von mittels Pinsel oder Abstreifern, Roll-on-Vorrichtungen oder Zerstäubern auftragbaren flüssigen Zusammensetzungen, als Stifte und in Form von aus normalen Flaschen und Behältern auftragbaren Systemen, z. B. Crèmes, Gelen oder Lotionen vorliegen. So können sie z. B. eine Lösung, eine Emulsion vom Typ Wasser-in-Öl (W/O) oder vom Typ Öl-in-Wasser (O/W), oder eine multiple Emulsion, beispielsweise vom Typ Wasser-in-Öl-in-Wasser (W/O/W), Öl-in-Wasser-in-Öl (O/W/O), ein Gel, eine Hydrodispersion, eine lamellare Phase, eine flüssige isotrope Lösungsphase, eine micellare Phase, eine solide oder dispergierte ein- oder mehrfach hexagonale Phase, eine solide oder dispergierte ein- oder mehrfach kubische Phase, eine lyotrope Phase, eine kristalline Phase, einen festen Stift oder auch ein Aerosol darstellen.

Weiterhin können die erfindungsgemäßen Zubereitungen bzw. Verwendungen vorteilhaft in Form von Gesichtswässern, Tinkturen, Reinigungsformulierungen, Pads, Wattebäuschen oder Tüchern sowie in Form von Tonics oder Shampoos vorliegen.

Vorteilhaft wird der pH-Wert der erfindungsgemäßen Zubereitungen bzw. Verwendungen im schwach sauren bis neutralen Bereich eingestellt, bevorzugt von 3,0 - 7,0, besonders bevorzugt von 4,0 - 6,5.

Die erfindungsgemäßen Formulierungen können wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden oder in den kosmetischen und dermatologischen Reinigungsprodukten.

Es ist dem Fachmann natürlich bekannt, dass kosmetische Zubereitungen zumeist nicht ohne die üblichen Hilfs- und Zusatzstoffe denkbar sind. Die erfindungsgemäßen kosmetischen und dermatologischen Zubereitungen können dementsprechend ferner kosmetische Hilfsstoffe enthalten, wie sie üblicherweise in solchen Zubereitungen verwendet werden; beispielsweise Konsistenzgeber, Konservierungsmittel, Stabilisatoren, Füllstoffe, Parfüme, Pigmente, die färbende Wirkung haben, Verdickungsmittel, Suspendiermittel, Puffergemische, oberflächenaktive Substanzen, Emulgatoren, weichmachende, anfeuchtende und/oder feuchthaltende Substanzen, entzündungshemmende Substanzen, zusätzliche Wirkstoffe wie Vitamine oder Proteine, Lichtschutzmittel, Insektenrepellentien, Bakterizide, Wasser, Salze, antimikrobiell, proteolytische oder keratolytisch wirksame Substanzen, Medikamente oder andere übliche Bestandteile einer kosmetischen oder dermatologischen Formulierung wie Alkohole, Polyole, Polymere, Schaumstabilisatoren, organische Lösungsmittel oder auch Elektrolyte.

Die jeweils einzusetzenden Mengen an Trägerstoffen können in Abhängigkeit von der Art des jeweiligen Produktes vom Fachmann durch einfaches Ausprobieren leicht ermittelt werden.

Als übliche Trägerstoffe zur Herstellung der erfindungsgemäßen Zubereitungen bzw. Verwendungen können neben Wasser, Ethanol und Isopropanol, Glycerin und Propylenglykol hautpflegende Fett- oder fettähnliche Stoffe, wie Ölsäuredecylester, Cetylalkohol, Cetylstearylalkohol und 2-Octyldodecanol, in den für solche Präparate üblichen Mengenverhältnissen eingesetzt werden, sowie schleimbildende Stoffe und Verdickungsmittel, z. B. Hydroxyethyl- oder Hydroxypropylcellulose, Polyacrylsäure, Polyvinylpyrrolidon, daneben aber auch in kleinen Mengen cyclische Silikonöle (Polydimethylsiloxane) sowie flüssige Polymethylphenylsiloxane niedriger Viskosität.

Als Emulgatoren zur Herstellung der erfindungsgemäßen Zubereitungen bzw. Verwendungen, welche vorteilhaft als flüssige oder feste Zubereitungen auf die gewünschten Hautbereiche aufgetragen werden sollen, und die in den Zubereitungen in geringer Menge, z.B. 1 bis 6 Gew.-%, bezogen auf die Gesamt-Zusammensetzung, verwendet werden können, haben sich nichtionogene Typen, wie Polyoxyethylen-Fettalkoholether, z. B. Cetostearylalkoholpolyethylenglykolether mit 12 bzw. 20 angelagerten Ethylenoxid-Einheiten pro Molekül, Cetostearylalkohol sowie Sorbitanester und Sorbitanester-Ethylenoxid-Verbindungen (z. B. Sorbitanmonostearat und Polyoxyethylensorbitanmonostearat) und langkettige höhermolekulare wachsartige Polyglykolether als geeignet erwiesen. Daneben sind aber auch eine ganze Reihe von anderen Emulgatoren oder Emulgatormischungen geeignet, welche üblicherweise in kosmetischen Zubereitungen Verwendung finden. Dazu zählen beispielsweise, aber nicht beschränkend auf, Glycerylstearatcitrat, PEG-40 Stearat oder auch Polyglyceryl(3)-Methylglucosedistearat, Stearinsäure, Steareth-2 und Steareth-21.

Die Ölphase der erfindungsgemäßen Zubereitungen wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Verbindungen der Ölphase können synthetischer, halbsynthetischer oder natürlicher Herkunft sein.

Auch beliebige Abmischungen solcher Öl- und Wachskomponenten sind vorteilhaft im Sinne der vorliegenden Erfindung einzusetzen.

Vorteilhaft beträgt der Gehalt an der Ölphase zwischen 1 und 50 Gew.-%, bezogen auf das Gesamtgewicht der Zubereitungen, bevorzugt 2 - 30 Gew.-%, insbesondere bevorzugt unter 10 Gew.-%.

Zur Anwendung werden die erfindungsgemäßen kosmetischen und/oder dermatologischen Formulierungen in der für Kosmetika und Dermatika üblichen Weise auf die Haut, Kopfhaut und/oder die Haare in ausreichender Menge aufgebracht.

### Lipidassay zur Testung der Wirksamkeit erfindungsgemäßer TOFA-Derivate

Für die Bestimmung des Lipid-reduzierenden Effekts von Wirkstoffen werden primäre Zelllinien von Talgdrüsen unterschiedlicher menschlicher Spender verwendet. Die Zellen werden für insgesamt 7 Tage kultiviert. Nachdem die Zellen an Tag 1 ausgesät werden, erfolgt die Wirkstoffbehandlung an Tag 3 und Tag 5. An Tag 7 werden die Zellen mit dem Farbstoff AdipoRed angefärbt. Dieser ermöglicht es die Lipidtröpfchen im Inneren der Zellen fluoreszent anzufärben und zu quantifizieren. Zur Kontrolle der Vitalität der Zellen wird außerdem der Farbstoff Fluoresceindiacetat (FDA) verwendet, der von lebenden Zellen umgesetzt wird. Das entstehende Fluoreszenzsignal kann ebenfalls quantifiziert werden. Der Effekt eines Wirkstoffes kann als Ratio des AdipoRed und FDA Fluoreszenzsignals relativ zu der Ratio des entsprechenden Lösungsmittels gemessen und dargestellt werden. Die getesteten Substanzen wurden in Dimethylsulfoxid (DMSO) gelöst, dementsprechend wurden alle Ergebnisse auf die mit DMSO behandelte Kontrolle normiert.

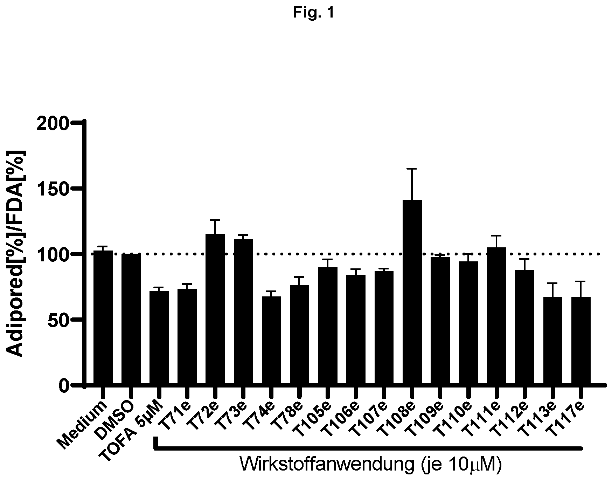

Fig. 1 zeigt die Ergebnisse des Assays.

Die folgenden Beispiele sollen die Erfindung erläutern, aber nicht einschränken. Die Zahlenangaben beziehen sich auf Gew.-%, sofern nichts anderes angegeben ist.

| | Beispiel 1 | Beispiel 2 | Beispiel 3 | Beispiel 4 |
|---|---|---|---|---|
| Caprylic/Capric Triglyceride | | | | |
| Propylene Glycol + Aqua | | | 5,00 | 5,00 |
| Citric Acid | | 0,00 | 0,00 | 0,00 |
| Cetearyl Alcohol | 2,00 | | | |
| Aqua + Trisodium EDTA | 1,00 | | | |
| Phenoxyethanol + Aqua | 0,40 | | | |
| Dimethicone | 3,00 | | | |
| Glycerin | 6,00 | 4,00 | 4,00 | 10,00 |
| PEG-40 Hydrogenated Castor Oil | | | 1,00 | 5,00 |
| Butylene Glycol + Aqua | | 20,00 | 10,00 | 10,00 |
| Hydroxyethylcellulose + Disodium Phosphate + Sodium Phosphate | 0,15 | | | |
| Alcohol Denat. + Aqua | 8,00 | 50,00 | 40,00 | 40,00 |
| Xanthan Gum | 0,15 | | | |
| Acrylates/C10-30 Alkyl Acrylate Crosspolymer | 0,15 | | | |
| PEG-150 Distearate | 0,50 | | | |
| Tapioca Starch + Aqua | 1,00 | | | |
| Ammonium Acryloyldimethyltaurate/VP Copolymer + Aqua | 0,50 | 0,65 | 0,65 | 0,65 |
| Methylpropanediol | | | 4,00 | 4,00 |
| Sodium Stearoyl Glutamate + Sodium Chloride | 0,30 | | | |
| | | | | |
| Tofa-Derivat T 105 e | 2,00 | | | |
| Tofa-Derivat T 71 e | | 1,00 | | |
| Tofa-Derivat T 74 e | | | 2,00 | |
| Tofa-Derivat T 113 e | | | | 2 |
| | | | | |
| | | | | |
| | | | | |
| **Wasser auf** | **100,00** | **100,00** | **100,00** | **100,00** |
| | | | | |

| | Beispiel 5 | Beispiel 6 | Beispiel 7 | |
|---|---|---|---|---|
| Caprylic/Capric Triglyceride | | | 4,00 | |
| Propylene Glycol + Aqua | 5,00 | | | |
| Citric Acid | 0,00 | | | |
| Cetyl Alcohol | | | 3,00 | |
| Aqua + Trisodium EDTA | | 1,00 | 1,00 | |
| Diisopropyl Adipate | | 10,00 | 10,00 | |
| Phenoxyethanol + Aqua | | 0,50 | 0,80 | |
| Hydrogenated Coco-Glycerides | | | 2,00 | |
| Glycerin | 10,00 | 3,00 | 9,00 | |
| Aqua + Sodium Hydroxide | | 0,06 | 0,01 | |
| PEG-40 Hydrogenated Castor Oil | 5,00 | | | |
| Butylene Glycol + Aqua | 10,00 | | 5,00 | |
| Alcohol Denat. + Aqua | 40,00 | 10,00 | 5,00 | |
| Xanthan Gum | | 0,30 | 0,30 | |
| Acrylates/C10-30 Alkyl Acrylate Crosspolymer | | | 0,20 | |
| Glyceryl Stearate Citrate | | | 2,00 | |
| Acrylates/C10-30 Alkyl Acrylate Crosspolymer | | 0,20 | | |
| Ammonium Acryloyldimethyltaurate/VP Copolymer + Aqua | 0,65 | | | |
| Methylpropanediol | 4,00 | | | |
| Tofa-Derivat T 105 e | 0,50 | | | |
| Tofa-Derivat T 113 e | | 0,50 | | |
| Tofa-Derivat T 117 e | | | 1 | |
| | | | | |
| | | | | |
| **Wasser auf** | **100,00** | **100,00** | **100,00** | |
| | | | | |

## Patentansprüche

1. Verbindung, **dadurch gekennzeichnet, dass** sie gewählt werden aus der Gruppe

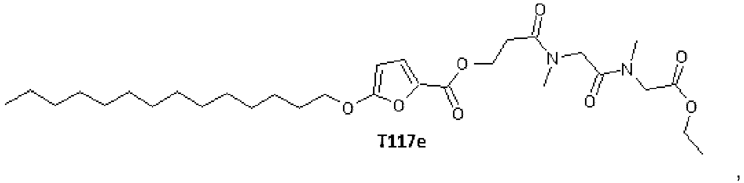

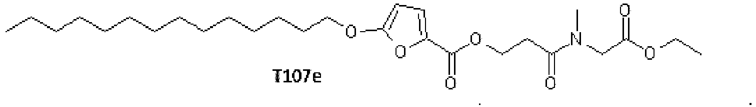

2. Medizinische oder kosmetische topische Zubereitungen, enthaltend eine oder mehrere Verbindungen gemäß Anspruch 1 in einer wirksamen Konzentration,

3. Medizinische oder kosmetische topische Zubereitungen, enthaltend eine oder mehrere Verbindungen gemäß Anspruch 1 in Konzentrationen von 0,0001 -40,00 Gew.-%, bevorzugt 0,005 - 20,00 Gew.-%, besonders bevorzugt 0,001 - 6,00 Gew.-%, jeweils bezogen auf das Gesamtgewicht der Zusammensetzung, vorliegen.

4. Kosmetische, nicht therapeutische Verwendung einer oder mehrerer Verbindungen gemäß Anspruch 1 zur Verringerung der Sebumproduktion in der menschlichen Haut,

5. Eine oder mehrere Verbindungen gemäß Anspruch 1 zur therapeutischen Behandlung von milden Formen der Akne und/oder der Seborrhö.

## Claims

1. Compound, **characterized in that** they are selected from the following group:

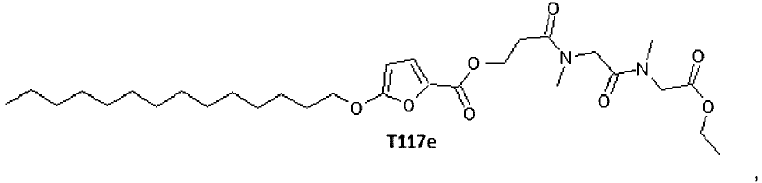

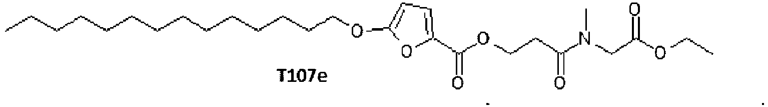

2. Medicinal or cosmetic topical preparations containing one or more compounds according to Claim 1 at an effective concentration,

3. Medicinal or cosmetic topical preparations containing one or more compounds according to Claim 1 at concentrations of 0.0001 - 40.00% by weight, preferably 0.005 - 20.00% by weight, particularly preferably 0.001 - 6.00% by weight, in each case based on the total weight of the composition.

4. Cosmetic, non-therapeutic use of one or more compounds according to Claim 1 for reducing sebum production in human skin,

5. One or more compounds according to Claim 1 for the therapeutic treatment of mild forms of acne and/or seborrhoea.

## Revendications

1. Composé, **caractérisé en ce qu'**il est choisi dans le groupe

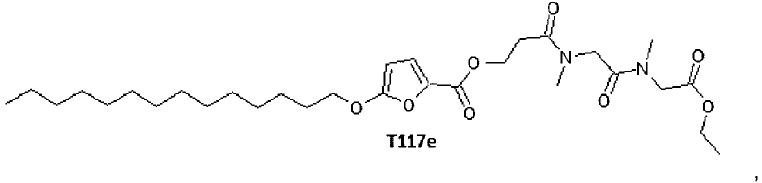

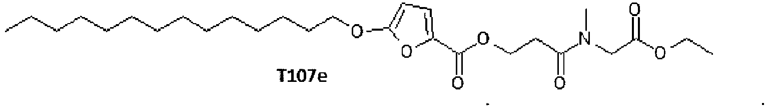

2. Préparations topiques médicales ou cosmétiques, contenant un ou plusieurs composés selon la revendication 1 en une concentration efficace.

3. Préparations topiques médicales ou cosmétiques, contenant un ou plusieurs composés selon la revendication 1 en des concentrations de 0,0001 à 40,00 % en poids, de préférence 0,005 à 20,00 % en poids, de manière particulièrement préférée 0,001 à 6,00 % en poids, à chaque fois par rapport au poids total de la composition.

4. Utilisation cosmétique, non thérapeutique, d'un ou plusieurs composés selon la revendication 1 pour diminuer la production de sébum dans la peau humaine.

5. Composé(s) selon la revendication 1 pour le traitement thérapeutique de formes légères de l'acné et/ou de la séborrhée.
